# EUROPEAN PATENT APPLICATION

(11) **EP 4 792 737 A2**
(43) Date of publication of application: **19.08.2026**
(21) Application number: 26189747.4
(22) Date of filing: 21.05.2024
(51) Int. Cl.: A61B 5/0205

(54) **DETECTION DEVICE, DETECTION SYSTEM, AND MODEL GENERATION DEVICE**

(30) Priority: 30.05.2023 JP 2023088969
(62) Document number(s) of the earlier application(s) in accordance with Art. 76 EPC: 26156087.4 / 4 714 339; 24177154.2 / 4 470 453
(71) Applicant: FCL Components Limited, Tokyo 140-0002 (JP)
(72) Inventor: EBATA, Kazuyoshi, Shinagawa-ku, 140-0002 (JP)
(74) Representative: Haseltine Lake Kempner LLP

(57) **Abstract**

A detection device includes a sensor configured to detect first information on motion of a human body of a user, and a processor configured to generate fourth information based on second information on biological activity of the user and third information on biological activity of the user acquired from another detection device, and transmit the fourth information to a server, the second information being generated based on the first information.

## Description

### FIELD

A certain aspect of the embodiments is related to a detection device, a detection system, and a model generation device.

### BACKGROUND

There is known a device for confirming the safety of a user by using a microwave Doppler sensor. There is known a system for watching over or confirming the safety of a user in a toilet or a bathroom.

In the case of monitoring the state of the user in each of the plurality of compartments, when each of a plurality of detection devices in the same compartment communicates with a server, the communication load becomes large. Note that the technique related to the present disclosure is disclosed in Patent Document 1 (Japanese Laid-Open Patent Publication No. 2012-75861), Patent Document 2 (Japanese Laid-Open Patent Publication No. 2021-149220), Patent Document 3 (Japanese Laid-Open Patent Publication No. 2016-218773), and Patent Document 4 (Japanese National Publication of International Patent Application No. 2022-547258).

### SUMMARY

It is an object of the present disclosure to provide a detection device, a detection system, and a model generation apparatus are provided in which a communication load is reduced.

According to a first aspect of the present disclosure, there is provided a detection device including: a sensor configured to detect first information on motion of a human body of a user; and a processor configured to generate fourth information based on second information on biological activity of the user and third information on biological activity of the user acquired from another detection device, and transmit the fourth information to a server, the second information being generated based on the first information.

According to a second aspect of the present disclosure, there is provided a detection system including: a plurality of first detection devices, wherein one of the plurality of first detection devices is installed in each of a plurality of compartments, and acquires first information on biological activity of a user in a corresponding compartment; and a plurality of second detection devices, wherein one or more second detection devices are provided in each of the plurality of compartments, and acquire second information on biological activity of the user in a corresponding compartment; wherein each of the plurality of first detection devices acquires the second information from the one or more second detection devices installed in a same compartment among the plurality of second detection devices, generate third information based on the first information and the second information, and transmit the third information to a server.

According to a third aspect of the present disclosure, there is provided a model generation device including: a memory; and a processor coupled to the memory and the processor configured to: acquire a plurality of pieces of information outputted from a plurality of detection devices, each of the plurality of detection devices being installed in each of a plurality of compartments and detecting information on biological activity of a user in a corresponding compartment; generate a model for determining a state of the user in each of the plurality of compartments based on the plurality of pieces of information; and transmit the model to the plurality of detection devices.

According to a fourth aspect of the present disclosure, there is provided a detection device including: a sensor configured to transmit a first electromagnetic wave, receive a second electromagnetic wave that is a reflected wave of the first electromagnetic wave reflected at an object, and generate an analog signal relating to motion of the object based on the first electromagnetic wave and the second electromagnetic wave; a processor configured to convert the analog signal into a digital signal and generate information on the motion of the object based on the digital signal; and an adjuster configured to adjust an amplitude of the analog signal based on the digital signal and output an adjusted analog signal to the processor.

According to a fifth aspect of the present disclosure, there is provided a detection device including: a sensor configured to transmit a first electromagnetic wave, receive a second electromagnetic wave that is a reflected wave of the first electromagnetic wave reflected at an object, and generate information on the object based on the first electromagnetic wave and the second electromagnetic wave; a window through which the first electromagnetic wave and the second electromagnetic wave pass; and a heater configured to suppress condensation on the window by heating the window.

According to an embodiment of the present disclosure, the communication load can be reduced.

### BRIEF DESCRIPTION OF DRAWINGS

FIG. 1 is a block diagram of a detection device according to a first embodiment.
FIG. 2 is a block diagram illustrating a detection system in the first embodiment.
FIGs. 3A and 3B are plan views illustrating an example of a compartment in the first embodiment.
FIGs. 4A and 4B are plan views illustrating an example of a compartment in the first embodiment.
FIG. 5 is a block diagram of a server in the first embodiment.
FIG. 6 is a sequence diagram in the first embodiment.
FIG. 7 is a flowchart of processing executed by a processing unit of the detection device according to the first embodiment.
FIG. 8A is a diagram illustrating a voltage with respect to a time for a signal 26e in the first embodiment.
FIG. 8B is a table illustrating a signal 26f.
FIGs. 9A to 9C are tables illustrating information D10a to D10c in the first embodiment, respectively.
FIG. 10 is a table illustrating information D12 in the first embodiment.
FIG. 11 is a flowchart of processing executed by a processor of a server in the first embodiment.
FIG. 12 is a sequence diagram in the first embodiment.
FIG. 13 is a flowchart of processing executed by the processing unit of the detection device according to the first embodiment.
FIGs. 14A to 14C are tables illustrating information D16, D20, and D22 in the first embodiment, respectively.
FIG. 15 is a flowchart of processing executed by a processor of a server in the first embodiment.
FIG. 16 is a flowchart illustrating processing executed by the processing unit of the detection device according to the first embodiment.
FIG. 17 is a schematic diagram of a vehicle according to a second embodiment.
FIG. 18 is a sequence diagram in a second embodiment.
FIG. 19 is a cross- sectional view of a detection device according to a third embodiment.

### DESCRIPTION OF EMBODIMENTS

Hereinafter, a description will be given of the embodiment of the present disclosure with reference to the drawings.

Facilities such as hotels and other accommodations, hospitals, schools, police, and public institutions are divided into a plurality of compartments. One or more users are staying in each of the compartments. For example, in a hotel, the plurality of compartment correspond to a plurality of rooms. In the hotel, each room has a toilet and a bathroom. Therefore, if only one detection device for detecting the biometric information of the user is provided in one room, the biometric information of the user may not be detected when the user is in the toilet or the bathroom. Therefore, it is conceivable to install a plurality of detection devices in one room. When a plurality of detection devices are set in one room, if a plurality of pieces of information of the plurality of detection devices in the plurality of rooms are individually transmitted to the server, a communication load becomes large.

In the following embodiment, only one detection device in a plurality of detection devices installed in one room communicates with the server. This can reduce the load of communication between the detection device and the server.

### [First Embodiment]

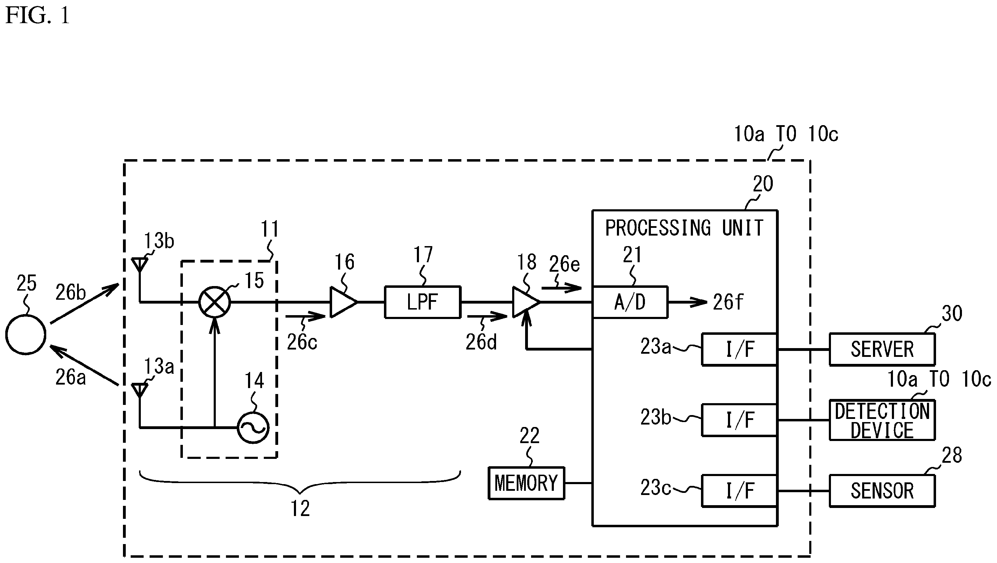

FIG. 1 is a block diagram of a detection device according to a first embodiment. Each of the detection devices 10a to 10c includes a sensor 12, a PGA (Programmable Gain Amplifier) 18, and a memory 22. The sensor 12 includes an antenna 13a for transmission, an antenna 13b for reception, a high frequency circuit 11, an amplifier 16, and a LPF (Low Pass Filter) 17. The high frequency circuit 11 includes an oscillator 14 and a mixer 15. The high frequency circuit 11 is provided with an amplifier or the like, but the description thereof is omitted. The antennas 13a and 13b are, for example, patch antennas provided on a substrate. The antenna 13a transmits a signal 26a generated by the oscillator 14 to a user 25 or the like. The antenna 13b receives a signal 26b reflected by the user 25 when the signal 26a is irradiated to the user 25. The signals 26a and 26b are electromagnetic waves, for example, microwaves or millimeter waves. The frequency of the signal 26a is, for example, 10 GHz to 120 GHz, and for example, around 24 GHz. The mixer 15 mixes the signals 26a and 26b and outputs a converted signal 26c. The frequency of the signal 26c output by the mixer 15 corresponds to a difference between the frequency of the signal 26a and the frequency of the signal 26b. Thus, the signal 26c becomes an analog signal corresponding to the movement of the user 25 in a range irradiated with the signal 26a.

The amplifier 16 amplifies the signal 26c. The LPF 17 suppresses a signal having a frequency higher than that of the signal of the biological vibration in the amplified signals 26c, passes a signal having a frequency (for first embodiment0 Hz or less) of the biological vibration, and outputs a filtered signal 26d. The PGA 18 amplifies the signal 26d and outputs the amplified signal as the signal 26e. The signal 26e is an analog signal mainly including biological information in the analog signal corresponding to the movement of the user 25.

A processing unit 20 is, for example, a processor such as a CPU (Central Processing Unit) or a microcomputer, and executes processing in cooperation with software. The processing unit 20 includes an A/D (Analog-Digital) converter 21 and interfaces (I/F) 23a to 23c. The A/D converter 21 converts the analog signal 26e into the digital signal 26f. The I/F 23a transmits and receives information to and from a server 30. The I/F 23b transmits and receives information from other detection devices 10a to 10c. The I/F 23c transmits and receives information to and from a sensor 28. The detection devices 10b and 10c in the detection devices 10a to 10c may not be provided with the I/Fs 23a and 23c. The processing unit 20 generates information such as the heart rate, the respiratory rate, the body motion, and the heart rate variability of the user 25 based on the signal 26f. The processing unit 20 determines the state of the user from the generated information such as the heart rate, the respiratory rate, the body motion or the heart rate variability. The memory 22 is a nonvolatile memory or a volatile memory, and stores setting conditions for performing processing, data in the process of calculating information, programs, and the like.

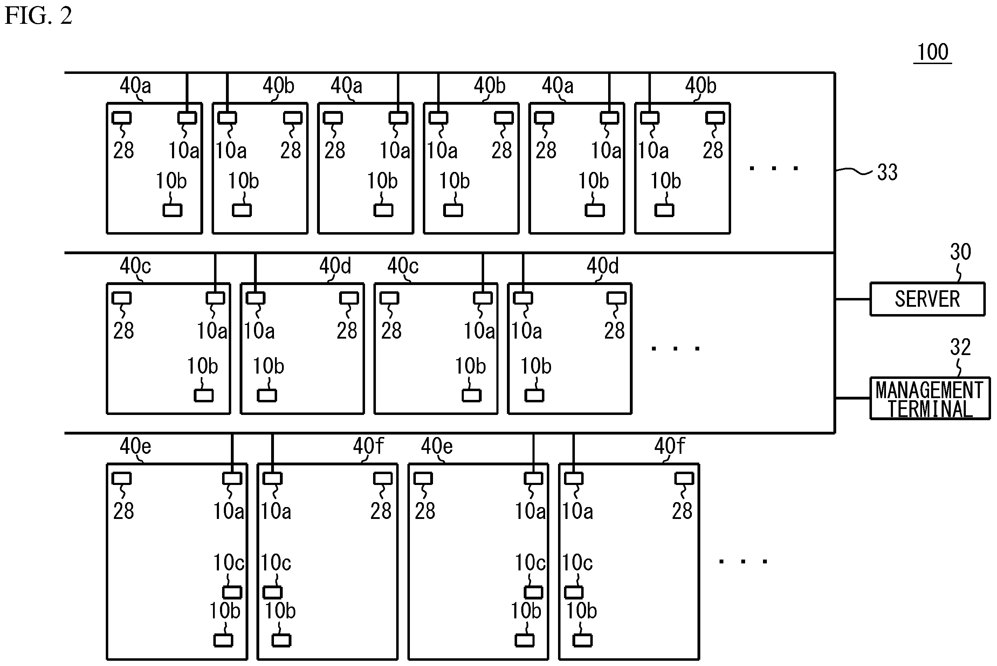

FIG. 2 is a block diagram illustrating a detection system according to the first embodiment. A plurality of compartments 40a to 40f are provided in the facility. In a detection system 100, a plurality of detection devices 10a and 10b are installed in each of the plurality of compartments 40a to 40d, and a plurality of detection devices 10a to 10c are installed in each of the plurality of compartments 40e and 40f. The sensor 28 is provided in each of the plurality of compartments 40a to 40f. The compartments 40a to 40f correspond to, for example, rooms of an accommodation facility. The compartments 40a and 40b are left-right symmetric rooms in the accommodation facility, the compartments 40c and 40d are left-right symmetric rooms, and the compartments 40e and 40f are left-right symmetric rooms.

The detection device 10a corresponds to a master device, and the detection devices 10b and 10c correspond to slave devices. The sensor 28 detects indicators such as temperature, humidity, illuminance, sound level, sound frequency, opening and closing of doors, and/or on/off of lighting switches for each of the compartments 40a to 40f. A plurality of sensors for detecting different indicators may be provided in each of the compartments 40a to 40f. The detection devices 10a, 10b, 10c, and the sensor 28 in each of the compartments 40a to 40f are connected by wireless or wired communication such as Wi-Fi (registered trademark) or Bluetooth (registered trademark). The detection devices 10a of the compartments 40a to 40f are connected to the server 30 and a management terminal 32 via a network 33. The network 33 is a wireless or wired network, such as LAN (Local Area Network) or wireless LAN.

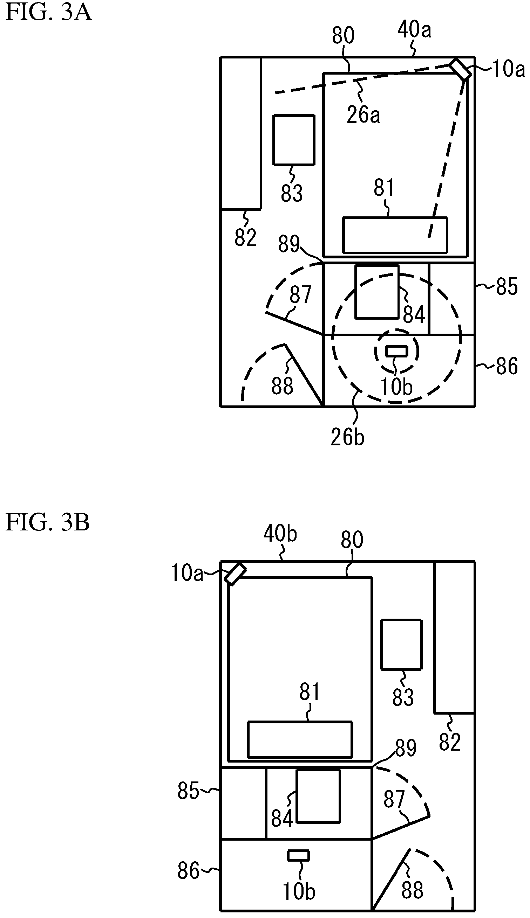

FIGs. 3A to 4B are plan views illustrating examples of the compartment in the first embodiment. The compartments 40a and 40b in FIGs. 3A and 3B are examples of single rooms. A bed 80, a pillow 81, a desk 82 and a chair 83 are set in the compartments 40a and 40b. The bed 80 is, for example, a single bed. A toilet seat 84, a washstand 85, and a bathtub 86 are provided in a bathroom 89. The bathroom 89 is a semi-compartment serving as a toilet and a bathroom. A door 87 is provided in the bathroom 89, and a door 88 is provided in each of the compartments 40a and 40b. The detection device 10a is located at one corner of each of the compartments 40a and 40b, and the detection device 10b is located in the bathroom 89. If only one detection device 10a is provided in the compartment 40a and the detection device 10a is installed at a corner of the compartment 40a, the signal 26a is spread throughout the compartment 40a. However, when the user is in the bathroom 89, the door 87 is closed, or a water droplet is attached to the curtain or the like, the detection device 10a may not be able to acquire the biometric information of the user. Therefore, the detection device 10b is installed in the bathroom 89. Thus, the signal 26b transmitted by the detection device 10b is spread throughout the bathroom 89 in the compartment 40a. The compartment 40a and the compartment 40b are symmetrical rooms.

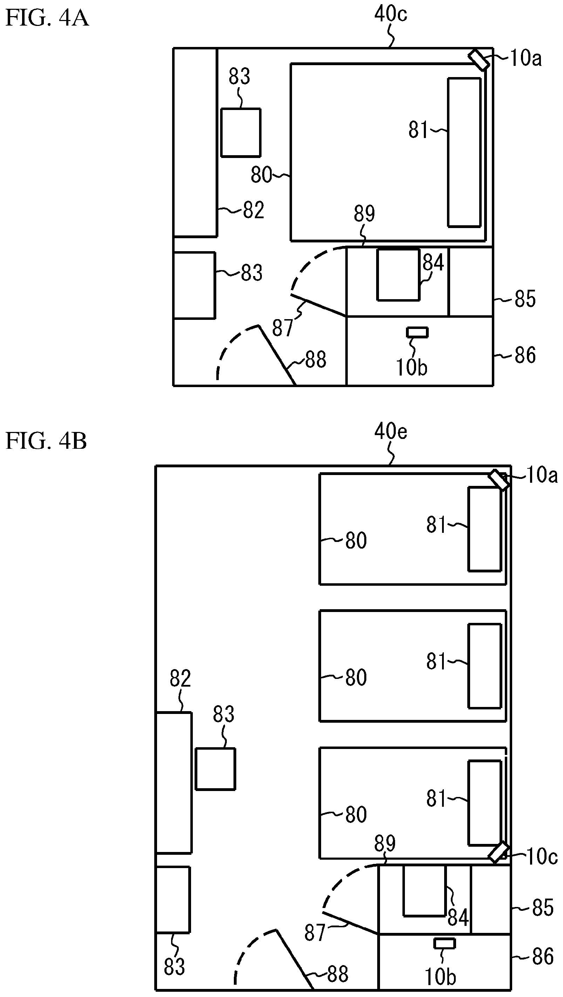

The compartment 40c in FIG. 4A is an example of a double room. In the compartment 40c, the bed 80 is larger than the bed 80 in the compartments 40a and 40b, and is, for example, a double bed. The chair 83 is provided beside the desk 82. Thus, the area of the compartment 40c is larger than the areas of the compartments 40a and 40b. The detection device 10a is installed at a corner of the compartment 40c, and the detection device 10b is provided in the bathroom 89. The compartment 40d in FIG. 2 is a compartment that is symmetrical with respect to the compartment 40c. In accommodations such as hotels or living facilities such as apartments, there are many rooms with a structure and arrangement symmetrical with respect to a wall.

The compartment 40e in FIG. 4B is an example of a triple room. In the compartment 40e, three beds 80 are provided. Therefore, the area of the compartment 40e is larger than the area of the compartment 40c. Since the area of the compartment 40e is large, three detection devices 10a to 10c are provided in the compartment 40e. The compartment 40f in FIG. 2 is a compartment that is symmetrical with respect to the compartment 40e.

The compartment may be a unit of a patient room in a hospital, a unit of a classroom in a school, or a unit of a room or a seat in a police station and a public institution, for example.

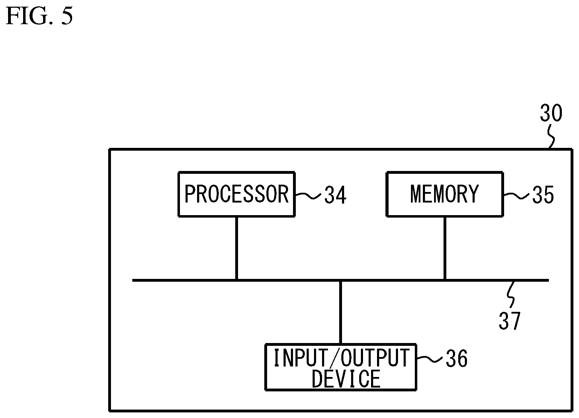

FIG. 5 is a block diagram of the server in the first embodiment. The server 30 includes a processor 34, a memory 35, an input/output device 36, and an internal bus 37. The processor 34 is, for example, a CPU, and executes processing such as generation of a model. The memory 35 is, for example, a volatile memory or a nonvolatile memory, and stores data and the like used when the processor 34 executes the processing. The memory 35 may store programs executed by the processor 34. The input/output device 36 inputs data and information acquired by the processor 34 from an external device, and outputs data output by the processor 34 to the external device. The internal bus 37 connects the processor 34, the memory 35, and the input/output device 36 to each other, and transmits data and the like. The server 30 cooperates with the software to grasp the situation of each of the compartments 40a to 40f and also functions as a model generation device.

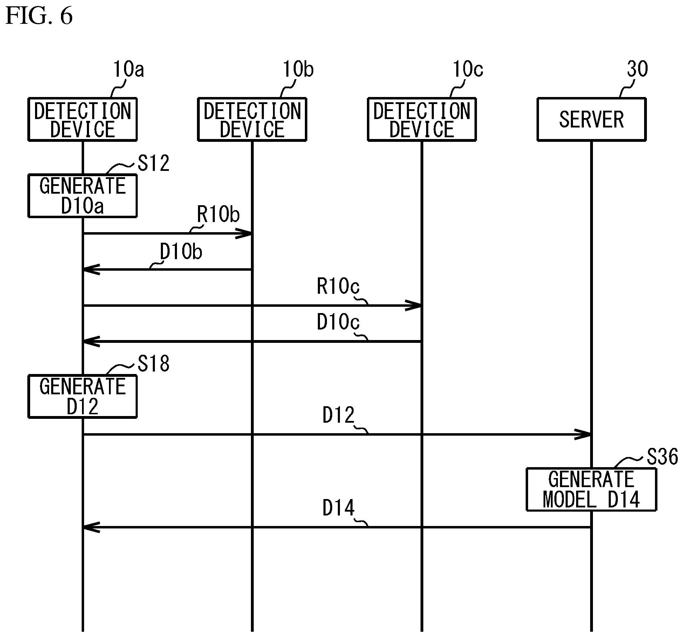

First, the processing of each device when the server 30 grasps the situation of the compartments 40a to 40f and generates a model will be described. FIG. 6 is a sequence diagram of the first embodiment. Three detection devices 10a to 10c are installed in a single compartment. The detection device 10a generates information D10a (S12). Next, the detection device 10a issues an information request R10b to the detection device 10b. The detection device 10b transmits information D10b on the biological activity of the user acquired by the detection device 10b to the detection device 10a. Next, the detection device 10a issues an information request R10c to the detection device 10c. Next, the detection device 10c transmits information D10c on the biological activity of the user acquired by the detection device 10c to the detection device 10a.

Next, the detection device 10a generates information D12 based on the plurality of pieces of information D10a to D10c (S18). Next, the detection device 10a transmits the information D12 to the server 30. The server 30 receives the information D12 from the detection devices 10a of the plurality of compartments 40a to 40f, and stores the information D12 in the memory 35. Next, the server 30 generates a model D14 for the detection device 10a to determine the state of the user based on the accumulated information D12 while referring to a compartment or the like having a similar structure (S36). Next, the server 30 transmits the generated model D14 to the detection device 10a, and the detection device 10a receives the model D14. In the example of FIG. 2, the compartments having similar structures are the compartments 40a to each other, the compartments 40b to each other, the compartments 40c to each other, the compartments 40d to each other, the compartments 40e to each other, and the compartments 40f to each other.

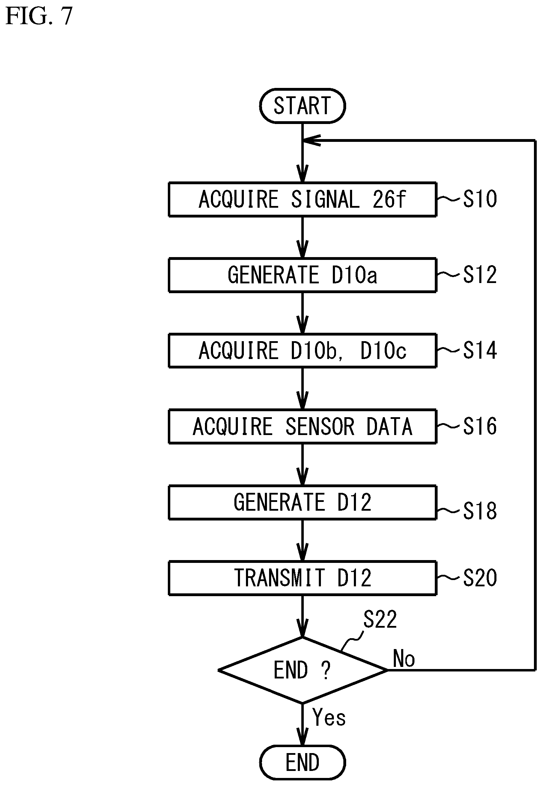

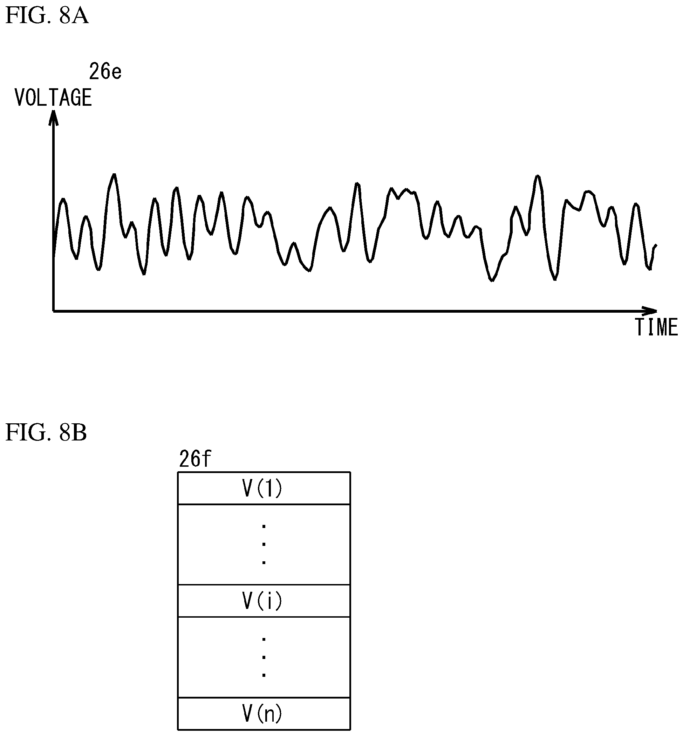

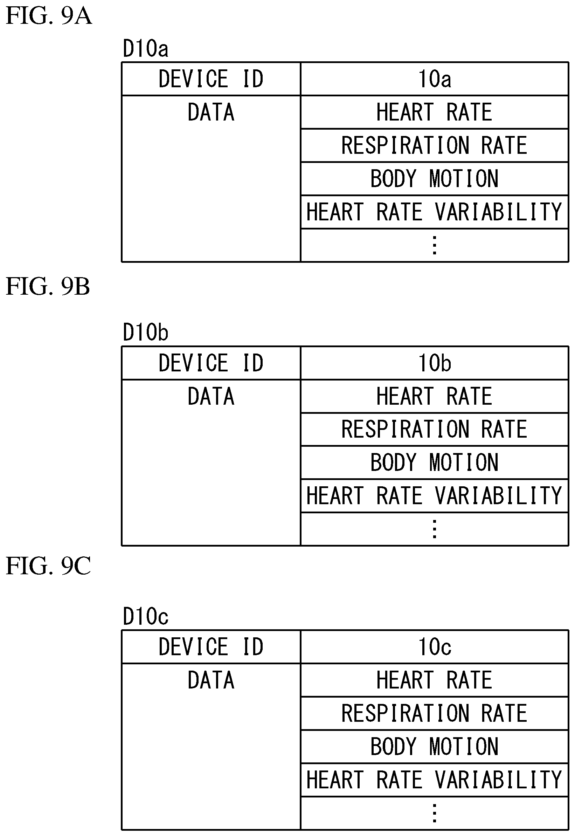

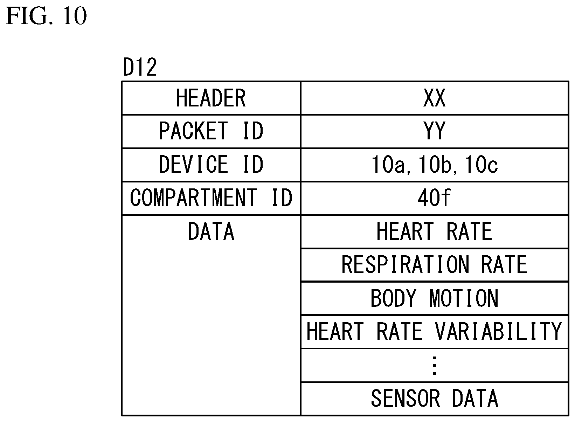

FIG. 7 is a flowchart of processing executed by the processing unit of the detection device according to the first embodiment. FIG. 8A is a diagram illustrating a voltage with respect to a time for the signal 26e in the first embodiment. FIG. 8B is a table illustrating the signal 26f. FIGs. 9A to 9C are tables illustrating the information D10a to D10c in the first embodiment, respectively. FIG. 10 is a table illustrating the information D12 in the first embodiment.

As illustrated in FIG. 7, the processing unit 20 of the detection device 10a acquires the signal 26f (S10). The acquisition of the signal 26f will be described. The A/D converter 21 acquires the signal 26e from the PGA 18. As illustrated in FIG. 8A, the signal 26e is an analog signal of voltage with respect to time, and in the signal 26e, a waveform corresponding to the biological information of the user such as heartbeat and respiration is superimposed on a waveform component indicating the motion of the body of the user. The A/D converter 21 converts the analog signal 26e into the digital signal 26f. As illustrated in FIG. 8B, the signal 26f is a voltage V(1)..., V(i)..., V(n). The symbol i is an integer of 1 to n, and corresponds to the time illustrated in FIG. 8A. That is, the signal 26f is information in which the plurality of voltages V(i) are arranged in time series. For example, when a sampling interval of the A/D converter 21 is t, a time interval between the voltages V(i) and V(i+1) is t.

Next, the processing unit 20 generates the information D10a based on the signal 26f (S12). As illustrated in FIG. 9A, the information D10a stores "10a" as a device ID, and "heart rate", "respiration rate", "body motion", "heart rate variability", and the like as data. The device ID is an identification code indicating the detection device that acquired the signal 26e. The "heart rate" is data corresponding to the heart rate of the user for one minute. The "respiration rate" is data corresponding to the respiration rate of the user for one minute. The "body motion" is a motion of the body of the user other than the heartbeat and the respiration, and is, for example, the number and the intensity of motions for one minute. The "Heart rate variability" corresponds to the variability of heart rate. These data are generated by Fourier-transforming the signal 26f and then analyzing the Fourier-transformed signal by the processing unit 20.

As illustrated in FIGs. 6 and 7, the processing unit 20 acquires the information D10b from the detection device 10b and acquires the information D10c from the detection device 10c (S14). As illustrated in FIGs. 9B and 9C, the information D10b and D10c store "10b" and "10c" as the device ID, respectively, and each of the information D10b and D10c stores "heart rate", "respiration rate", "body motion", "heart rate variability", and the like as data. The processing units 20 of the detection devices 10b and 10c generate the information D10b and D10c, respectively, as in S10 and S12 of FIG. 7, and transmit the information D10b and D10c to the detection device 10a based on the requests R10b and R10c of FIG. 6.

As illustrated in FIG. 7, the processing unit 20 acquires sensor data from the sensor 28 (S16). The sensor data is data indicating, for example, temperature, humidity, illuminance, sound level, sound frequency, opening and closing of the door, and/or on/off of the lighting switch. The order of executing S10, S12, S14, and S16 can be set as appropriate.

Next, the processing unit 20 generates the information D12 based on the plurality of pieces of information D10a to D10c and the sensor data (S18). As illustrated in FIG. 10, the information D12 stores "XX" as a header, "YY" as a packet ID, "10a", "10b" and/or "10c" as device IDs, and "40f" as a compartment ID, and stores "heart rate", "respiration rate", "body motion", "heart rate variability", "sensor data", and the like as data. The header is a header of a packet transmitted by the server 30, and the packet ID is an identification code indicating the packet. The device ID is an identification code of the detection device that acquired the signal 26e. The compartment ID is an identification code of the detected compartment. The compartment ID may be a MAC (Media Access Control) address or an IP address (Internet Protocol) of the detection device 10a of each compartment. When one of the detection devices 10a to 10c mainly detects the biometric information of the user, the processing unit 20 includes, in the information D12, one of the plurality of pieces of information D10a to D10c corresponding to the one detection device that has detected the biometric information of the user.

As illustrated in FIG. 7, the processing unit 20 transmits the information D12 to the server 30 (S20). The communication between the processing unit 20 and the server 30 may be encrypted. Next, the processing unit 20 determines whether to end the processing (S22). For example, when the server 30 instructs the end of the processing, the processing unit 20 determines that the determination of S22 is Yes, and otherwise determines that the determination of S22 is No. If the determination of S22 is No, the processing returns to step S10. If the determination of S22 is yes, the processing ends.

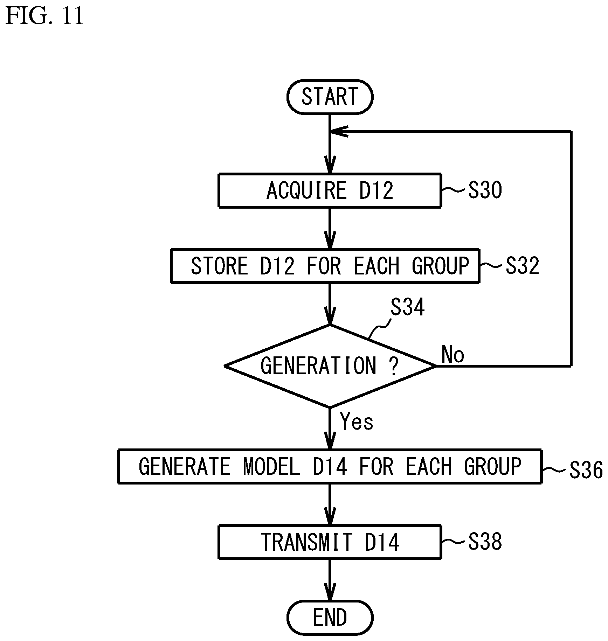

FIG. 11 is a flowchart of processing executed by the processor of the server in the first embodiment. First, the processor 34 acquires information D12 corresponding to each of the compartments 40a to 40f from each of the detection devices 10a installed in the plurality of compartments 40a to 40f in FIG. 2 (S30). Next, the processor 34 stores the information D12 in the memory 35 for each group (S32). For example, in FIG. 2, the plurality of compartments 40a is a single group, and the plurality of compartments 40b is a single group different from the group of the compartments 40a. Similarly, the plurality of compartments 40a, the plurality of compartments 40b, the plurality of compartments 40c, the plurality of compartments 40d, the plurality of compartments 40e, and the plurality of compartments 40f are different groups from each other, respectively. At this time, information on the state of the user is stored in the memory 35 in association with each of the plurality of pieces of information D12. The state of the user is, for example, a state in which the user is relaxed, a state in which the user is sleeping, a state in which the user is nervous, a state in which the user is abnormal, or the like. The information on the state of the user may be included in the information D12 or may be acquired from another source.

Next, the processor 34 determines whether to generate a model (S34). For example, the processor 34 determines that the determination of S34 is Yes when a predetermined period of time has elapsed or when an instruction to generate the model is given from an external device, and determines that the determination of S34 is No in other cases. If the determination of S34 is No, the processing returns to S30, and the information D12 is accumulated in S30 and S32.

If the determination of S34 is Yes, the processor 34 generates the model D14 for each group based on the information D12 (S36). For example, the processor 34 generates the model D14 by machine learning in which the information D12 of each group and the information on the state of the user when the information D12 is acquired are used as teacher data. The teacher data may include sensor data in the information D12, information on dates such as weekdays and holidays or days of the week, information on time zones, and/or the number of users in the compartment. For example, even if the heart rate of the user is the same, the user is likely to be asleep when the illuminance is low or at midnight, and is likely to be relaxed when the illuminance is high or until noon. Next, the processor 34 transmits the model D14 generated for each group to the detection devices 10a of the compartments 40a to 40f included in the corresponding group (S38). Then, the processing ends.

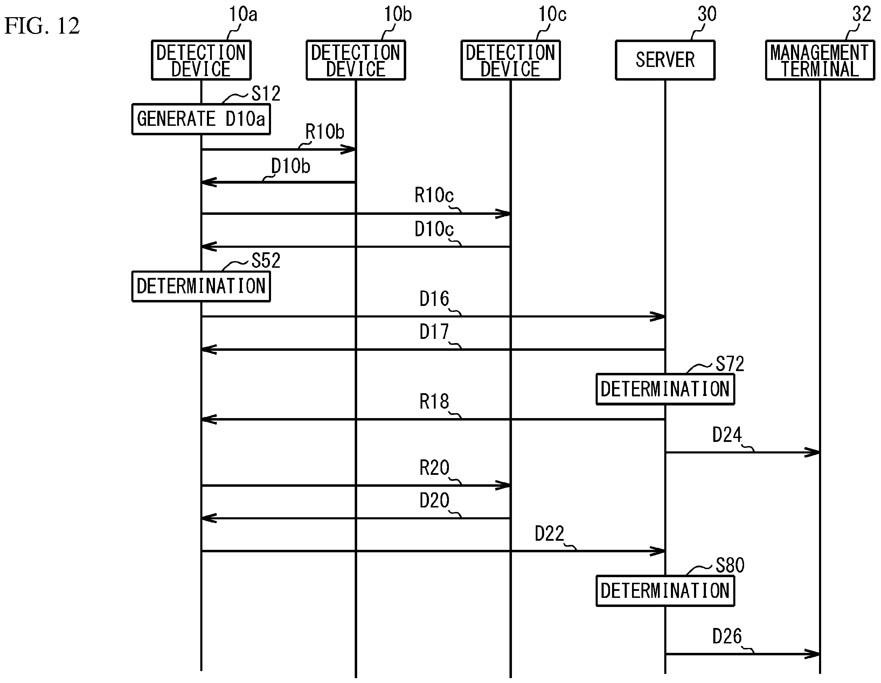

The processing of each device when the detection device 10a determines the state of the user by using the model will be described. FIG. 12 is a sequence diagram of the first embodiment. Three detection devices 10a to 10c are installed in a single compartment. As in FIG. 6, the detection device 10a generates the information D10a (S12). The detection device 10a receives the information D10b and D10c from the detection devices 10b and 10c, respectively.

Next, the detection device 10a determines the state of the user based on the information D10a to D10c using the model (S52). When the detection device 10a determines that the user (or the compartment) is abnormal, the detection device 10a transmits information D16 indicating the abnormality to the server 30. In this case, the information D16 indicating abnormality may be, for example, information including no data or including information indicating "abnormality" with a small information amount. Upon receiving the information D16, the server 30 transmits information D17 for reception confirmation to the detection device 10a. The detection device 10a may transmit the information D16 a plurality of times until receiving the information D17. In this case, when the detection device 10a receives the information D17, the detection device 10a stops the transmission of the information D16. This makes it possible to suppress the information D16, which is important information, from not reaching the server 30 even if the network 33 is congested. Next, the server 30 determines whether to request detailed information based on the information D16 (S72). When the server 30 determines that the detailed information is to be requested, the server 30 transmits a request R18 for detailed information to the detection device 10a. Next, the server 30 transmits attention information D24 to the management terminal 32. An administrator operating the management terminal 32 recognizes that attention is required.

Next, the detection device 10a transmits a request R20 for detailed information to the detection device 10c that has detected the biometric information of the user. Next, the detection device 10c transmits detailed information D20 to the detection device 10a. Next, the detection device 10a transmits information D22 including the information D20 to the server 30. Next, the server 30 determines whether the user (or the compartment) is abnormal based on the information D22 (S80). When the server 30 determines that the user (or the compartment) is abnormal, the server 30 transmits the information D26 of the abnormality to the management terminal 32. The administrator operating the management terminal 32 recognizes that an abnormality has occurred. The management terminal 32 or the server 30 may send an alarm to a portable terminal such as a smartphone of a person who notifies the abnormality, and notify the abnormality.

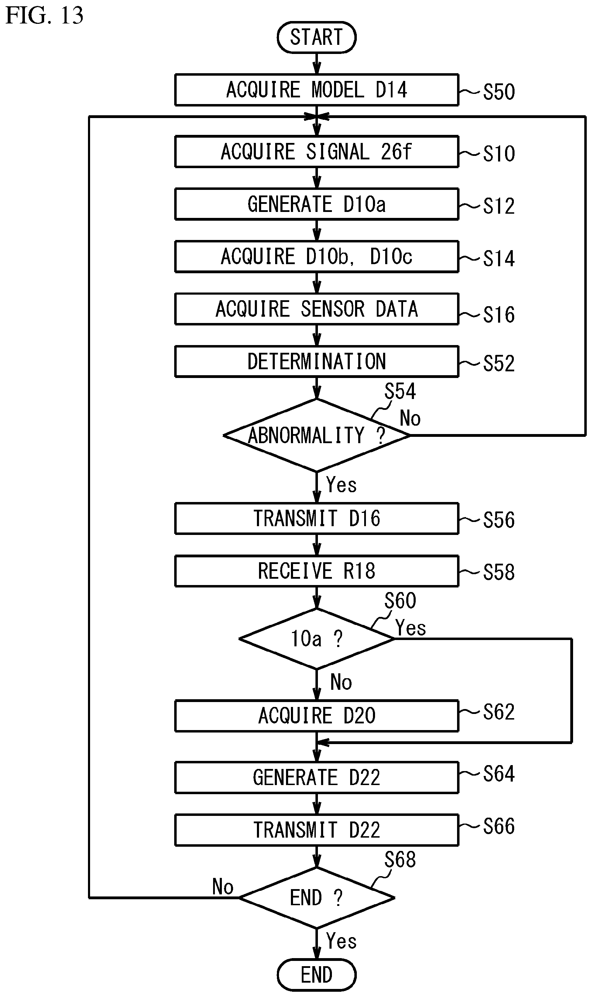

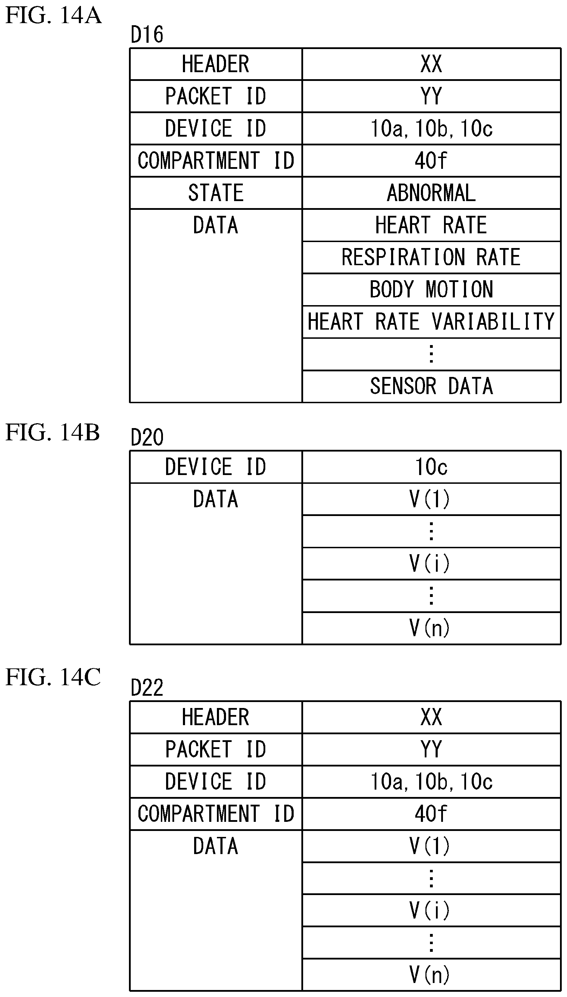

FIG. 13 is a flowchart of processing executed by the processing unit of the detection device according to the first embodiment. FIGs. 14A to 14C are tables illustrating information D16, D20, and D22 in the first embodiment, respectively.

As illustrated in FIG. 13, the processing unit 20 of the detection device 10a acquires the model D14 from the server 30 (S50). Next, the processing unit 20 executes S10, S12, S14, and S16 as in FIG. 7. Next, the processing unit 20 determines the state of the user (or the compartment) based on the information D10a to D10c and the sensor data (S52). The model D14 is the model generated in step S36 of FIG. 11. For example, when the information D10a to D10c and the sensor data are input to the model, the processing unit 20 can determine the state of the user (or the compartment). Next, the processing unit 20 determines whether the state of the user (or the compartment) is abnormal (S54). If the determination of S54 is No, the processing returns to S10.

If the determination of S54 is Yes, the processing unit 20 transmits the information D16 to the server 30 (S56). As illustrated in FIG. 14A, the information D16 stores "XX" as the header, "YY" as the packet ID, "10a", "10b" and/or "10c" as the device ID, "40f" as the compartment ID, "abnormal" as the state, and "heart rate", "respiration rate", "body motion", "heart rate variability", "sensor data", and the like as data. The information D16 includes the state of the user in addition to the information D12 of FIG. 10. The device ID "10c" indicates that the detection device 10c has detected the biometric information of the user. The state of the user "abnormal" indicates that the state of the user (or the compartment) determined by using the model D14 is "abnormal". The information D16 may not include data, but may be only information indicating that the user (or the compartment) is abnormal. When the information D16 includes urgent information, the server 30 transmits the information D17 to the detection device 10a upon receiving the information. The detection device 10a sends a packet including the information D16 a plurality of times until the detection device 10a receives the information D17. This makes it possible to ensure that the information D16 reaches the server 30 even if the packet loss of the information D16 occurs. The urgent information D16 is, for example, information indicating that the heartbeat or respiration of the user whose heartbeat has been measured has stopped. The detection device 10a may continue to send the information D16 to the server 30 as a short packet a limited number of times by using a connectionless protocol such as UDP (User Datagram Protocol).

Referring back to FIG. 13, the processing unit 20 receives the request R18 from the server 30 (S58). Next, the processing unit 20 determines whether the detailed information of the detection device 10a is requested (S60). If the determination of S60 is Yes, the processing proceeds to S64. If the determination of S60 is No, the processing unit 20 transmits the request R20 to the detection device that requests detailed information in the detection devices 10b and 10c, and acquires the information D20 from the detection device 10b or 10c (S62). As illustrated in FIG. 14B, the information D20 stores "10c" as the device ID and V(1), V(i)..., and V(n) as the data. The V(i) corresponds to the signal 26f (see FIG. 8B) acquired by the processing unit 20 of the detection device 10c.

Referring back to FIG. 13, the processing unit 20 generates the information D22 (S64). As illustrated in FIG. 14C, the information D22 stores "XX" as the header, "YY" as the packet ID, "10a", "10b", and/or "10c" as device IDs, and "40f" as the compartment ID, and stores V(1)..., V(i)... and V(n) as data. When the data V(i) of the detection device 10a is transmitted, the data V(i) corresponds to the signal 26f acquired in S10. When the data V(i) of the detection device 10b or 10c is transmitted, the data V(i) corresponds to the information D20 acquired in S62.

As illustrated in FIG. 13, the processing unit 20 transmits the information D22 to the server 30 (S66). Next, the processing unit 20 determines whether to end the processing (S68). If the determination of S68 is No, the processing returns to step S10. If the determination of S68 is yes, the processing ends.

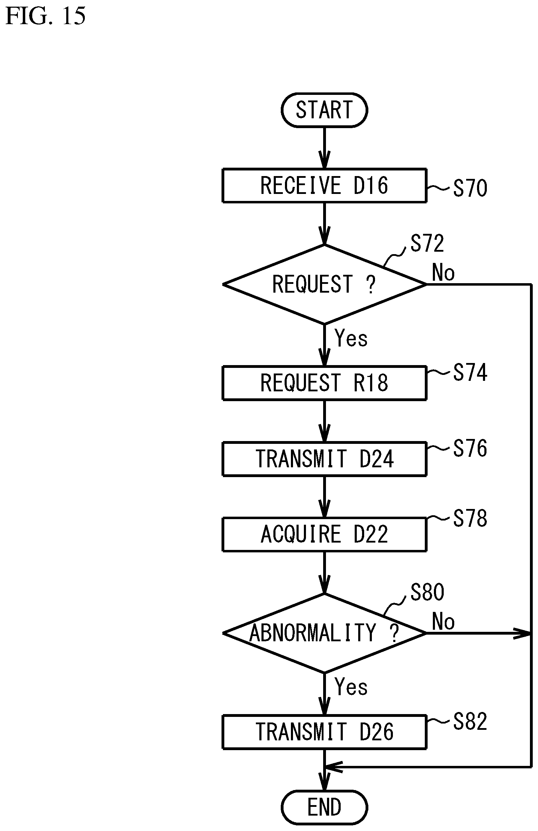

FIG. 15 is a flowchart of processing executed by the processor of the server in the first embodiment. First, the processor 34 acquires the information D16 from the detection device 10a (S70). Next, the processor 34 determines whether the request for detailed information is necessary based on the information D16 (S72). For example, when the information D16 is received from the detection device 10a of the compartment where the user is not supposed to be, the processor 34 determines that the request for detailed information is unnecessary. If the determination of S72 is No, the processing ends. S72 is not performed and the process may proceed to S74 when the information D16 is received.

If the determination of S72 is Yes, the processor 34 transmits the request R18 for detailed information to the detection device 10a (S74). Next, the processor 34 transmits the attention information D24 to the management terminal 32 (S76). The order of S74 and S76 may be reversed, or S76 may not be performed. Next, the processor 34 receives the detailed information D22 from the detection device 10a (S78). Next, the processor 34 determines whether the user (or the compartment) is abnormal based on the information D22 (S80). For example, the processor 34 compares the information of a certain compartment of the same group with the information of the other compartment of the same group, and when the detailed information of the certain compartment of the same group is similar to the detailed information of the other compartment of the same group, the processor 34 determines that the user is not abnormal. If the determination of S80 is No, the processing ends. If the determination of S80 is Yes, the processor 34 transmits the information D26 of the abnormality to the management terminal 32 (S82). Then, the processing ends.

When the detection devices 10a to 10c communicate with the server 30 via the network 33, the traffic of the network 33 increases, and the communication between the detection devices 10a to 10c and the server 30 becomes unstable.

Therefore, according to the first embodiment, one detection device 10a (first detection device) is installed in each of the plurality of compartments 40a to 40f, and one or a plurality of detection devices 10b and 10c (second detection device) are installed in each of the plurality of compartments 40a to 40f. As indicated by S10 of FIG. 7, the sensor 12 of the detection device 10a detects the signal 26f. The signal 26f is first information on the motion of the human body of the user in the compartment. As indicated by S14, the processing unit 20 acquires the information D10b and D10c from the detection devices 10b and 10c. The information D10b and D10c are third information on the biological activity of the user in the compartment. The processing unit 20 generates the information D10a (second information) relating to the biological activity of the user based on the signal 26f as indicated by S12, and further generates the information D12 (fourth information) based on the information D10a to D10c as indicated by S18. As indicated by S20, the processing unit 20 transmits the information D12 to the server 30 separately installed outside the compartment. In this way, the detection device 10a generates the information D12 to be transmitted to the server 30 based on the information D10b and D10c of the detection devices 10b and 10c. This makes it possible to suppress the traffic of the network 33 and reduce the communication load as compared with a case where the detection devices 10a to 10c transmit the plurality of pieces of information D10a to D10c to the server 30, respectively.

As illustrated in FIGs. 9A to 10, the processing unit 20 generates only a part of the plurality of pieces of information D10a to D10c as the information D12. This can further reduce the load of communication between the detection device 10a and the server 30.

The signal 26f is information in which values corresponding to at least a part of the motion of the human body of the user are arranged in time series. As illustrated in FIG. 10, the information D12 includes information corresponding to the heart rate and/or the respiration rate of the user, and does not include the signal 26f itself. In this way, the processing unit 20 does not transmit the signal 26f to the server 30, but transmits the heart rate, the respiration rate and the like of the user. This can reduce the load of communication between the detection device 10a and the server 30.

The processing unit 20 generates the information D10a (second information) corresponding to the heart rate and/or the respiration rate of the user based on the signal 26f as indicated by S12 of FIG. 7. This can reduce the load of communication between the detection device 10a and the server 30.

The detection devices 10b and 10c may transmit the data V(i) to the detection device 10a. However, in this case, the load of communication between the detection device 10a and the detection devices 10b and 10c increases. As illustrated in FIGs. 9B and 9C, the information D10b and D10c are information corresponding to the heart rate and/or the respiration rate of the user generated by the detection devices 10b and 10c. This allows the detection devices 10b and 10c to operate autonomously. Therefore, the load of communication between the detection device 10a and the detection devices 10b and 10c can be reduced. As illustrated in FIG. 10, the information D12 includes at least one of the plurality of pieces of information D10a to D10c, but does not include the signal 26f itself. This can reduce the load of communication between the detection device 10a and the server 30.

As illustrated in S30 of FIG. 11, the processor 34 of the server 30 acquires the plurality of pieces of information D12 transmitted by the detection devices 10a respectively installed in the plurality of compartments 40a to 40f. As indicated by S36, the processor 34 generates the models D14 for determining the states of the users in the plurality of compartments 40a to 40f based on the plurality of pieces of information D12. As indicated by S38, the processor 34 transmits the model D14 to the plurality of detection devices. As illustrated in S52 of FIG. 13, the processing unit 20 determines the state of the user based on the information D10a to D10c using the model D14 received from the server 30. In this way, the server 30 can generate the models D14 for determining the states of the users by using the plurality of pieces of information D12 of the compartments 40a to 40f as the teacher data. Therefore, the accuracy of the models D14 can be improved. When the detection device 10a in each of the compartments 40a to 40f generates the model, the load of the memory capacity of the detection device 10a and the load of the processing of the processing unit 20 increase, and the cost of the detection device 10a increases. The server 30 generates the models D14, thereby reducing the load of the detection device 10a.

As a comparative example, when the model is generated using the information D12 of the detection device 10a in one compartment as the teacher data, it takes time to collect the teacher data, and hence it takes time to generate the model D14. In the first embodiment, the model D14 can be generated using not only the information D12 of the detection device 10a in one compartment but also the plurality of pieces of information D12 of a large number of similar compartments as the teacher data. Therefore, more teacher data can be collected in a short period of time. Therefore, the highly accurate model D14 can be generated in a short period of time. The generated model D14 can be transmitted to the detection devices 10a of a large number of similar compartments via the network 33. Furthermore, the accuracy of the model D14 can be improved by adding information on the time, day of the week, and other sensors 28 to the teacher data.

As illustrated in FIGs. 2 to 4B, when the sizes of the rooms, the shapes of the rooms, and the arrangement of the equipment in the rooms are different in the compartments 40a to 40f, for example, the reflection states of the signals 26b are different from each other. For example, the reflection of the signal 26b may be weaker on furniture than on walls. Therefore, it may be better to use different models for determining the state of the user for each type of the compartments 40a to 40f. In the first embodiment, the plurality of compartments 40a to 40f are divided into the plurality of groups. The processor 34 generates the plurality of models D14 based on the plurality of pieces of information D12 transmitted by the detection devices 10a corresponding to the plurality of groups, and transmits the models D14 to the detection devices 10a of the corresponding groups, respectively. This makes it possible to generate the models suitable for the types of the compartments 40a to 40f, and to improve the accuracy of the models.

The rooms that are symmetrical, such as the compartments 40a and 40b, the compartments 40c and 40d, and the compartments 40e and 40f, may be grouped together or in groups of three. The rooms having the symmetrical shapes may be divided into six groups.

As illustrated in FIG. 13, the processing unit 20 transmits the signal 26f to the server 30 based on the instruction from the server 30 as indicated by S64 and S66. Alternatively, the processing unit 20 acquires the information D20 (fifth information) of FIG. 14B detected by the detection device 10b or 10c from the detection device 10b or 10c as indicated by S62. As indicated by S66, the processing unit 20 transmits the information D20 to the server 30. In this way, when the server 30 determines the abnormality of the user by using more detailed information, the detection device 10a can transmit the data V(i) to the server 30. The data amount of the data V(i) is very large. Therefore, as illustrated in FIGs. 6 and 7, the detection device 10a transmits the information D12 having a small data amount to the server 30 in the normal operation. This allows a margin in the load of communication between the plurality of compartments 40a to 40f and the server 30. As illustrated in FIGs. 12 and 13, when an abnormality is detected in one of the compartments 40a to 40f, even if the detection device 10a that has detected the abnormality transmits the information D22 having a large data amount to the server 30, the communication capacity can be suppressed from being exceeded.

The sensor 12 transmits the signal 26a (first electromagnetic wave) into the compartment, receives the signal 26b (second electromagnetic wave) in which the signal 26a is reflected by an object such as a user, and generates the signal 26e based on the signals 26a and 26b. In this way, by using a radar using the electromagnetic wave such a microwave or a millimeter wave, information on the motion of the user in the compartment can be detected with high accuracy. The sensor 12 may be a sensor other than a sensor using electromagnetic waves.

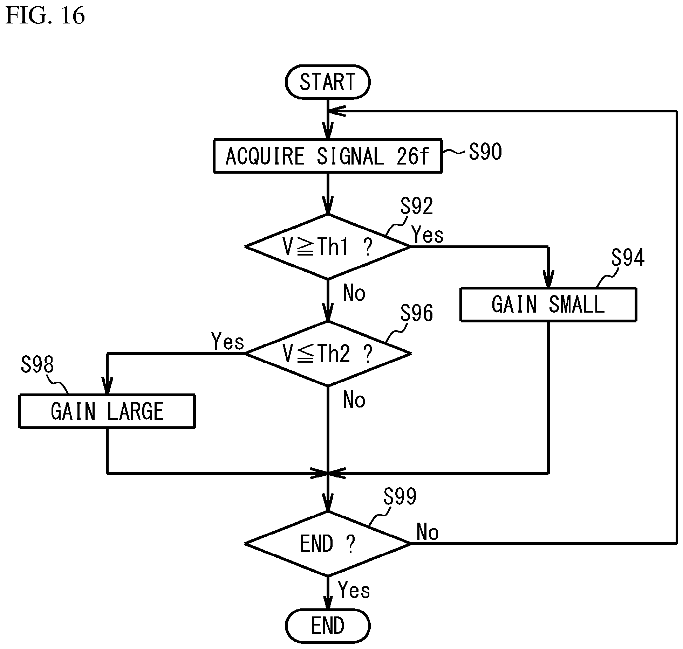

Next, a flow of the processing unit 20 adjusting the gain of the PGA 18 in FIG. 1 will be described. FIG. 16 is a flowchart illustrating processing executed by the processing unit of the detection device according to the first embodiment. The processing unit 20 acquires the signal 26f (S90). Next, the processing unit 20 determines whether the voltage V of the signal 26f is equal to or higher than a threshold value Th1 (S92). The processing unit 20 may compare a maximum value of the voltage V(i) (i is an integer from 1 to n) of the signal 26f with the threshold value Th1. The processing unit 20 may also determine that S92 is Yes when the number of voltages V(i) that are equal to or more than the threshold value Th1 is equal to or more than a predetermined number. Further, when the threshold value Th1 is set to the maximum value (for example, FFFF) of the data converted by the A/D converter 21 and V (i) which is the maximum value continues, it is considered that the A/D converter 21 is saturated. In such a case, the processing unit 20 may determine that S92 is Yes. If the determination of S92 is Yes, the processing unit 20 decreases the gain of the PGA 18 (S94). Then, the processing proceeds to S99.

If the determination of S92 is No, the processing unit 20 determines whether the voltage V is equal to or less than a threshold value Th2 (S96). The processing unit 20 may compare a minimum value of the voltage V(i) (i is an integer from 1 to n) of the signal 26f with the threshold value Th2. The processing unit 20 may also determine that S96 is Yes when the number of voltages V(i) that are equal to or less than the threshold value Th1 is equal to or more than a predetermined number. If the determination of S96 is No, the processing proceeds to S99. If the determination of S96 is Yes, the processing unit 20 increases the gain of the PGA 18 (S98). The processing proceeds to S99. Thereafter, the processing unit 20 determines whether to end the processing (S99). If the determination of S99 is No, the processing returns to step S90. If the determination of S99 is yes, the processing ends.

The sensor 12 generates the analog signal 26e relating to the biological activity of the user. The A/D converter 21 of the processing unit 20 converts the analog signal 26e into the digital signal 26f, and the processing unit 20 generates the information D10a on the biological activity of the user based on the digital signal 26f. In such detection devices 10a to 10c, for example, when the user approaches the detection devices 10a to 10c, the amplitude of the signal 26e increases. As a result, the analog signal 26e is clipped or saturated in the A/D converter 21, and is not normally converted into the digital signal. When the user is away from the detection devices 10a to 10c or is sleeping, the amplitude of the signal 26e reduces. This reduces the accuracy of the digital signal 26f. As illustrated in FIG. 16, the PGA 18 (adjuster) adjusts the amplitude of the analog signal 26e based on the digital signal 26f, and outputs the adjusted analog signal 26e to the processing unit 20. This makes it possible to increase the sensitivity of the sensor 12. The PGA 18 may be used in a detection device in which the sensor 12 generates the analog signal 26e relating to the motion of the object and the processing unit 20 generates information on the motion of the object.

### [Second Embodiment]

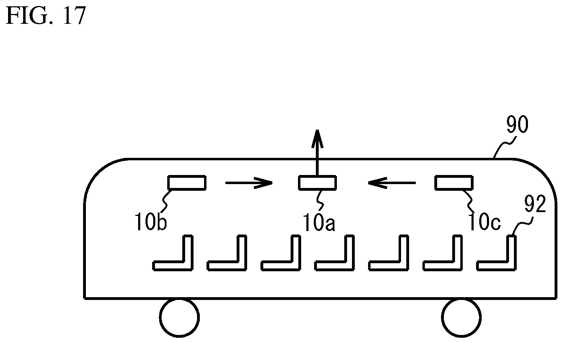

The second embodiment is an example in which the detection devices 10a to 10c are installed in a vehicle such as a school bus. FIG. 17 is a schematic diagram of the vehicle according to the second embodiment. A plurality of seats 92 are provided in a vehicle 90 such as a school bus. The plurality of detection devices 10a to 10c are provided on the ceiling of the vehicle 90. The detection device 10a is installed in the center of the vehicle 90, and the detection devices 10b and 10c are installed in the front side and the rear side of the vehicle, respectively. The detection device 10a and the detection devices 10b and 10c are connected by wireless or wired communication. The detection device 10a is connected to an external mobile terminal, for example, by wireless communication. The wireless system is, for example, a mobile communication system or Wi-Fi Direct (registered trademark). The structures of the detection devices 10a to 10c of the second embodiment are the same as those of the first embodiment.

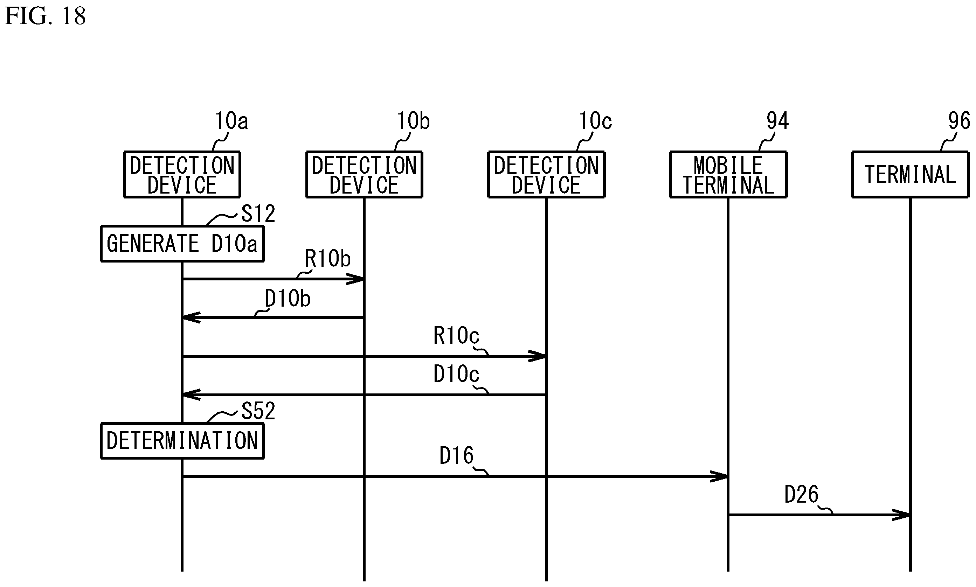

FIG. 18 is a sequence diagram in the second embodiment. The detection devices 10a to 10c, a mobile terminal 94, and a terminal 96 are provided. The mobile terminal 94 is a terminal capable of communicating with the detection device 10a, and is, for example, a cellular phone, a smart phone, or a tablet. The terminal 96 is a cellular phone, a smart phone, a fixed phone, or a personal computer, and is a terminal managed by an administrator. In FIG. 18, the flow of steps S12 to S52 is the same as that of FIG. 12 of the first embodiment, and the description thereof is omitted. When the detection device 10a determines that the user (or the vehicle 90) is abnormal in S52, the detection device 10a transmits information D16 indicating the abnormality to the mobile terminal 94. The mobile terminal 94 transmits information D26 indicating that there is an abnormality to the terminal 96. The administrator managing the terminal 96 recognizes that there is an abnormality in the vehicle 90. For example, the administrator can recognize abnormalities such as a user being left behind in the vehicle 90, a user being injured or ill, or a user being stuck.

As in the second embodiment, the detection devices 10a to 10c may be installed in vehicles other than buildings.

### [Third embodiment]

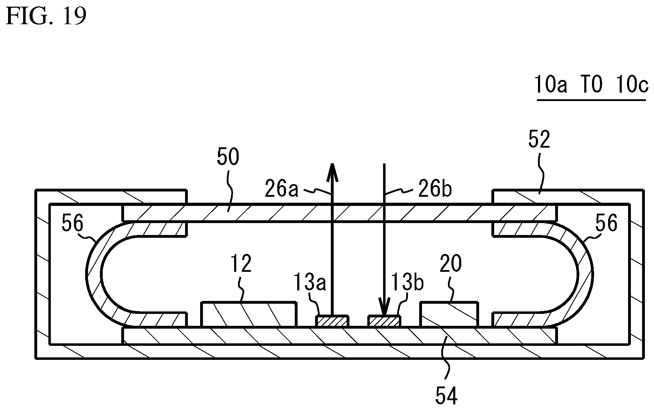

The third embodiment is an example of heating a window for allowing an electromagnetic wave output from an antenna to pass therethrough. FIG. 19 is a cross- sectional view of the detection devices 10a to 10c according to a third embodiment. A window 50 is provided in a housing 52. A substrate 54 is provided in the housing 52. The antennas 13a and 13b, the sensor 12, and the processing unit 20 are provided on the substrate 54. Heat conducting plates 56 are provided to thermally connect the substrate 54 and the window 50. The housing 52 is a metal plate made of, for example, stainless steel. The window 50 is a material that allows the signals 26a and 26b to pass therethrough and has good thermal conductivity, and is, for example, a heat-conductive resin plate. The heat conducting plate 56 is a metal plate having good thermal conductivity and, for example, a copper plate. The thermal conductivity of the heat conducting plate 56 is higher than the thermal conductivity of the housing 52, the window 50 and the substrate 54, for example. The other configurations of the detection devices 10a to 10c of the third embodiment are the same as those of the first embodiment.

In the third embodiment, when the detection device 10b is installed in the bathroom 89, water droplets or the like adhere to the window 50. Therefore, the signal 26a transmitted by the antenna 13a and the signal 26b received by the antenna 13b are less likely to pass through the window 50. Accordingly, the heat conducting plate 56 (heater) suppresses condensation on the window 50 by heating the window 50. This makes it possible to suppress the adhesion of water droplets or the like to the window 50. Accordingly, it is possible to suppress the signals 26a and 26b from being difficult to pass through the window 50.

A heater may be provided in the window 50. As in the third embodiment, by suppressing condensation on the window 50 by using the heat generated by the sensor 12, the power consumption associated with heating can be reduced. The heater may be provided in the detection device in which the sensor 12 generates the analog signal 26e relating to the motion of the object and the processing unit 20 generates information on the motion of the object.

All examples and conditional language provided herein are intended for the purposes of aiding the reader in understanding the invention and the concepts contributed by the inventor to further the art, and are not to be construed as limitations to such specifically recited examples and conditions, nor does the organization of such examples in the specification relate to a showing of the superiority and inferiority of the invention. Although one or more embodiments of the present invention have been described in detail, it should be understood that the various changes, substitutions, and alterations could be made hereto without departing from the spirit and scope of the invention.
The present invention extends to the following statements:
S1. A detection device (10a to 10c) comprising:
   a sensor (12) configured to detect first information on motion of a human body of a user; and
   a processor (20) configured to generate fourth information based on second information on biological activity of the user and third information on biological activity of the user acquired from another detection device, and transmit the fourth information to a server (30), the second information being generated based on the first information.
S2. The detection device according to statement S1, wherein
   the first information is information in which values corresponding to the motion of at least a part of the human body of the user are arranged in time series,
   the processor generates the second information corresponding to a heart rate and/or a respiration rate of the user based on the first information,
   the third information is information corresponding to a heart rate and/or a respiration rate of the user generated by the another detection device, and
   the fourth information includes at least one of the third information and the second information.
S3. The detection device according to statement S1 or statement S2, wherein
   the processor generates the fourth information based on the second information, the third information, and sensor data relating to installation environment acquired from a sensor (28).
S4. The detection device according to any of statements S1 to S3, wherein
   the processor transmits the first information to the server based on an instruction from the server, and/or
   the processor acquires fifth information in which values corresponding to the motion of at least a part of the human body of the user detected by the another detection device are arranged in time series from the another detection device, and transmits the fifth information to the server, based on the instruction from the server.
S5. The detection device according to any of the preceding statements, wherein
   the processor determines a state of the user based on the second information and the third information by using a model received from the server.
S6. A detection system (100) comprising:
   a plurality of first detection devices (10a), wherein one of the plurality of first detection devices is installed in each of a plurality of compartments (40a to 40f), and acquires first information on biological activity of a user in a corresponding compartment; and
   a plurality of second detection devices (10b and 10c), wherein one or more second detection devices are provided in each of the plurality of compartments, and acquire second information on biological activity of the user in a corresponding compartment;
   wherein each of the plurality of first detection devices acquires the second information from the one or more second detection devices installed in a same compartment among the plurality of second detection devices, generate third information based on the first information and the second information, and transmit the third information to a server.
S7. A model generation device (30) comprising:
   a memory (35); and
   a processor (34) coupled to the memory and the processor configured to:
      acquire a plurality of pieces of information outputted from a plurality of detection devices (10a to 10c), each of the plurality of detection devices being installed in each of a plurality of compartments (40a to 40f) and detecting information on biological activity of a user in a corresponding compartment;
      generate a model (D14) for determining a state of the user in each of the plurality of compartments based on the plurality of pieces of information; and
      transmit the model to the plurality of detection devices.
S8. The model generation device according to statement S7, wherein
   the plurality of compartments are divided into a plurality of groups, and
   the processor generates a plurality of models corresponding to the plurality of groups, respectively, based on a plurality of pieces of information output by the detection devices corresponding to the plurality of groups, and transmits the plurality of the models to the detection devices of the corresponding groups, respectively.
S9. A detection device (10a to 10c) comprising:
   a sensor (12) configured to transmit a first electromagnetic wave, receive a second electromagnetic wave that is a reflected wave of the first electromagnetic wave reflected at an object, and generate an analog signal relating to motion of the object based on the first electromagnetic wave and the second electromagnetic wave;
   a processor (20) configured to convert the analog signal into a digital signal and generate information on the motion of the object based on the digital signal; and
   an adjuster (18) configured to adjust an amplitude of the analog signal based on the digital signal and output an adjusted analog signal to the processor.
S10. A detection device (10a to 10c) comprising:
   a sensor (12) configured to transmit a first electromagnetic wave, receive a second electromagnetic wave that is a reflected wave of the first electromagnetic wave reflected at an object, and generate information on the object based on the first electromagnetic wave and the second electromagnetic wave;
   a window (50) through which the first electromagnetic wave and the second electromagnetic wave pass; and
   a heater (56) configured to suppress condensation on the window by heating the window.
S11. The detection device according to statement S10, wherein
   the heater suppresses the condensation on the window by using heat generated by the sensor.
A1. A detection system (100) comprising:
   a plurality of first detection devices (10a), wherein one of the plurality of first detection devices is installed in each of a plurality of compartments (40a to 40f), and acquires first biological activity information on biological activity of a user in a corresponding compartment, wherein the one of the plurality of first detection devices functions as a master device and has a first region in the corresponding compartment as a detection range; and
   a plurality of second detection devices (10b and 10c), wherein one or more second detection devices are provided in each of the plurality of compartments, and acquire second biological activity information on biological activity of the user in a corresponding compartment, wherein each of the one or more second detection devices as a slave device and has a second region different from the first region in the corresponding compartment as a detection range,
   wherein each of the plurality of first detection devices acquires the second biological activity information from the one or more second detection devices installed in a same compartment among the plurality of second detection devices, generate third biological activity information based on the first biological activity information and the second biological activity information, and transmit the third biological activity information to a server.
A2. The detection system according to statement A1, further comprising:
   the server (30);
   wherein the server includes:
      a memory (35); and
      a processor (34) coupled to the memory and the processor configured to:
         acquire a plurality of pieces of third biological activity information outputted from a plurality of first detection devices , each of the plurality of first detection devices being installed in each of a plurality of compartments (40a to 40f) and detecting information on biological activity of a user in a corresponding compartment;
         generate a model (D14) for determining a state of the user in each of the plurality of compartments based on the plurality of pieces of third biological activity information; and
         transmit the model to the plurality of first detection devices.
A3. The detection system according to statement A2, wherein
   the plurality of compartments are divided into a plurality of groups, and
   the processor generates a plurality of models corresponding to the plurality of groups, respectively, based on a plurality of pieces of third biological activity information output by the first detection devices corresponding to the plurality of groups, and transmits the plurality of the models to the first detection devices of the corresponding groups, respectively.
A4. The detection system according to statement A1,
   wherein each of the plurality of first detection devices (10a) includes:
   a sensor (12) configured to generate an analog signal relating to motion of the human body of the user;
   a processor (20) which includes an analog-to-digital converter configured to convert the analog signal into a digital signal, the processor generating the first biological activity information based on the digital signal; and
   an adjuster (18) configured to adjust an amplitude of the analog signal based on the digital signal and output an adjusted analog signal to the processor.
A5. The detection system according to statement A1,
   wherein each of the plurality of first detection devices (10a) includes:
   a sensor (12) which includes a transmission antenna configured to transmit a first electromagnetic wave and a reception antenna configured to receive a second electromagnetic wave that is a reflected wave of the first electromagnetic wave reflected by a human body of a user, the sensor detecting motion of the human body of the user based on the first electromagnetic wave and the second electromagnetic wave; and
   a window (50) through which the first electromagnetic wave and the second electromagnetic wave pass, and a heater (56) configured to suppress condensation on the window by heating the window.
A6. The detection system according to statement A5, wherein
   the heater suppresses the condensation on the window by using heat generated by the sensor.

## Claims

1. A detection device (10a) comprising:
a sensor (12) configured to detect motion of a human body of a user; and
a processing unit (20) configured to generate second information (D10a) relating to a biological activity of the user from first information (26f) relating to the motion of the human body of the user acquired based on an output of the sensor, generate fourth information (D12) based on the second information and third information (D10b, D10c) relating to the biological activity of the user acquired from one or more other detection devices (10b, 10c) separately installed, and transmit the fourth information to a server (30);
wherein the processing unit generates the second information corresponding to a heart rate and a respiration rate of the user based on the first information, and
the third information is information corresponding to the heart rate and the respiration rate of the user generated by the one or more other detection devices.

2. The detection device according to claim 1, wherein
the first information is information in which values corresponding to the motion of at least a part of the human body of the user are arranged in time series, and
the fourth information includes at least one of the second information and the third information.

3. The detection device according to claim 1, wherein
the processing unit generates the fourth information based on the second information, the third information, and sensor data relating to installation environment acquired from a sensor (28) separately installed.

4. The detection device according to claim 2, wherein
the processing unit is configured to, based on an instruction from the server:
transmit the first information to the server; and/or
acquire, from the one or more other detection devices, fifth information in which values corresponding to the motion of at least a part of the human body of the user detected by the one or more other detection devices are arranged in time series, and transmit the fifth information to the server.

5. The detection device according to claim 1, wherein
the processing unit determines a state of the user based on the second information and the third information by using a model received from the server.

6. The detection device according to claim 1, wherein
the sensor (12) includes a transmission antenna (13a) configured to transmit a first electromagnetic wave and a reception antenna (13b) configured to receive a second electromagnetic wave that is a reflected wave of the first electromagnetic wave reflected by the human body of the user, the sensor generating an analog signal relating to the motion of the human body of the user based on the first electromagnetic wave and the second electromagnetic wave,
the processing unit includes an analog-to-digital converter (21) configured to convert the analog signal into a digital signal, and
the detection device further includes an adjuster (18) configured to adjust an amplitude of the analog signal based on the digital signal and output the adjusted analog signal to the processing unit.

7. The detection device according to claim 1, wherein
the sensor (12) includes a transmission antenna (13a) configured to transmit a first electromagnetic wave and a reception antenna (13b) configured to receive a second electromagnetic wave that is a reflected wave of the first electromagnetic wave reflected by the human body of the user, and
the detection device further includes a window (50) through which the first electromagnetic wave and the second electromagnetic wave pass, and a heater (56) configured to suppress condensation on the window by heating the window.

8. The detection device according to claim 7, wherein
the heater suppresses the condensation on the window by using heat generated by the sensor.
